# EUROPÄISCHE PATENTANMELDUNG

(11) **EP 4 458 944 A1**
(43) Veröffentlichungstag der Anmeldung: **06.11.2024**
(21) Anmeldenummer: 24173843.4
(22) Anmeldetag: 02.05.2024
(51) Int. Cl.: C12M 1/42, C12M 1/00, C12M 1/12, C12M 1/26, C12M 1/34, C12N 1/02, C12N 1/12, C12N 13/00

(54) **VERFAHREN UND VORRICHTUNG ZUR KONTINUIERLICHEN ALGENPRODUKTION**

(30) Priorität: 02.05.2023 DE 102023111197
(71) Anmelder: Puevit GmbH, 01109 Dresden (DE)
(72) Erfinder: Mühlstädt, Gunnar, 01109 Dresden (DE); Krujatz, Felix, 01069 Dresden (DE)
(74) Vertreter: Dinter, Tilo

(57) **Zusammenfassung**

Die Erfindung betrifft die kontinuierliche Algenproduktion in einem Nährmedium innerhalb eines Reaktors unter Konstanthaltung der Biomassekonzentration bzw. der Trübung. Beschrieben wird ein Verfahren und eine dafür geeignete Anlage (sowie ihre Verwendung im Verfahren). Das Verfahren umfasst die Schritte:
1. Inokulation eines Nährmediums mit Algen,
2. Durchmischung bis zum Erreichen einer konstanten Biomassekonzentration von 0,3-0,8 g/L im Nährmedium,
3. Kontinuierlicher Betrieb der Algenkultivierung in dem Reaktor (1) unter Beibehaltung dieser Biomassekonzentration, umfassend die Schritte:
3a. Entfernen eines Teils des Nährmediums, sowie
3b. Abtrennung der Algen daraus, und
3c. Hinzufügen mindestens eines Teils des in Schritt 3a. entfernten Nährmediums nach Algenabtrennung als von Algen befreites, wieder aufbereitetes Nährmedium,
bei gleichzeitiger Regelung mindestens einer Regelgröße im kontinuierlichen Betrieb im Reaktor, ausgewählt aus Verdünnungsfaktor, eingestrahlte Lichtmenge und Temperatur, um die konstante Biomassekonzentration beizubehalten.

## Beschreibung

Die Erfindung betrifft die kontinuierliche Algenproduktion in einem Nährmedium innerhalb eines Reaktors unter Konstanthaltung der Biomassekonzentration bzw. der Trübung.

Algen sind vielfältig einsetzbar. Die technische Gewinnung von Algen spielt daher eine immer größer werdende Rolle. Dabei sind für geschlossene Photobioreaktoren verschiedene technologische Systeme verbreitet.

Bekannt ist die Algenkultivierung im Batchbetrieb, wie beispielsweise in WO2009/058621A1 beschrieben.

Andere Verfahren arbeiten im kontinuierlichen Betrieb.

In AT 5 171 88 A1 beispielsweise wird eine kontinuierliche Produktion in einem System mit horizontalen Verteilrohren und vertikalen Steig- und Fallrohren mit mäanderförmiger Strömung beschrieben, bei dem die Algenkonzentration bis auf einen sehr hohen Bereich von 10-50g/L anwächst. Die Algenabtrennung bei Erreichen dieses hohen Konzentrationsbereichs erfolgt mittels Filtration. Nachteil des Verfahrens ist aufgrund der sehr hohen Algenkonzentration eine schlechte volumetrische Produktivität der Algen. Gelegentlich verstopfen die Filter und erzeugen einen Wartungsaufwand.

EP 2 486 790 A1 beschreibt einen Rohrreaktor zur kontinuierlichen Mikroorganismenkultivierung mit CO₂-Einspeisung, wobei die Gasphase mindestens 0,2 Vol.-% CO₂ enthält. Die Fließgeschwindigkeiten betragen 0,2-0,3 m/s, maximal jedenfalls 0,5 m/s. Die Algenbiomasse gemäß erstem beschriebenem Experiment wächst dabei an von 0,1 g/L auf 3g/L, um möglichst viel Algen zu erhalten.

Einen Flachflächen-Photobioreaktor beschreibt WO 2010/002745 A1, welcher zur Untersuchung von Algenwachstumsmodellen mit entsprechender Vorhersagemöglichkeit genutzt wird.

US 2018 112165 A1 beschreibt einen Mikroalgenbioreaktor mit Turbiditätssensor, wobei im Batchbetrieb bis auf eine Algenkonzentration von 1g/L kultiviert wird um anschließend im Turbidostat-Betrieb (entspricht Konstanthalten der Trübung) mittels Turbiditätssensor die Verdünnung im Rahmen von 30% konstant zu halten, beispielsweise bei 0,5-2g/L. Die Algen werden mittels Filtration abgetrennt. Nachteilig kann es zur Verstopfungen der Filter kommen. Nachteil ist, dass die Bestimmung stark abhängig ist von der Zusammensetzung des Nährmediums, so dass die Fehlerquote bei der in-situ-Bestimmung der Algenkonzentration hoch ist. US 2018 112165 A1 legt nahe, die Algenkonzentration bevorzugt weitaus höher einzustellen, beispielsweise >1,0g/L.

Eine Alternative zum Turbidostat-Betrieb ist der sogenannte Chemostat-Betrieb, bei dem die Konzentration der Nährstoffe im Nährmedium konstant gehalten wird. Aufgrund einer nicht überwachten Algenkonzentration kommt es dabei allerdings trotz optimaler Nährstoffkonzentrationen oft zu schlechtem Wachstumsverhalten der Algen.

WO 2018/037402 A1 offenbart einen Bioreaktor zum Wachstum von Zellen und Mikroorganismen mit Rückführung des Kulturmediums und Trübungssensor sowie einer perforierten Barriere (Membrane) zwischen zwei Kammern des Bioreaktors zur Rückhaltung der Zellen bzw. Mikroorganismen in der einen, oberen Kammer. Der Aufbau ist aufwendig.

Hinsichtlich der Aufreinigung der sogenannten Kulturbrühen bei der Algenproduktion ist bekannt, im Batchbetrieb Charge für Charge einzeln bspw. mittels Zentrifugen von Algen zu befreien und diese Algen so zu sammeln um sie anschließend zu trocknen.

Im kontinuierlichen Betrieb dagegen wird vielfach die Ernte bzw. Abtrennung der Algen als "Flaschenhals"-Schritt beschrieben. Beispielsweise Lopez-Guzman et al. (2021) oder auch Visigalli et al. (2021) beschreiben, die Methoden Flotation und Elektrokoagulation zu kombinieren. Diese Kombination ist auch aus dem Bereich Abwasserbehandlung bei der Entfernung von Algen aus Abwasser bekannt, wie beispielsweise in US 2017/0113957 A1 beschrieben.

Viele der bekannten Methoden zur Algenproduktion (sowohl im Bereich Upstream d.h. bei der Kultivierung, als auch im Bereich Downstream d.h. bei der Abtrennung) sehen offene Arbeitsbereiche vor oder weisen aus anderen Gründen eine hohe Kontaminationsgefahr auf. Die gewonnenen Algen sind demnach nicht zum Verzehr geeignet bzw. der Prozess erfolgt nicht nach Standards (z.B. GMP).

Aufgabe der Erfindung ist es, die einfache und effektive Algenproduktion im großtechnischen Maßstab zu ermöglichen. Es soll in möglichst kurzer Zeit mit möglichst wenig Flüssigkeitsvolumen möglichst viel Algenbiomasse gewonnen werden können.

Das heißt unter anderem, es sollte mit der Erfindung möglich sein, die volumetrische Produktivität der Algen auf ein Maximum zu bringen. Die Methode und der Aufbau der Anlage sollten einfach sein, so dass auch im großtechnischen Maßstab eine kostengünstige Algenproduktion möglich wird. Das heißt auch, mit der Erfindung sollten Wartungsarbeiten verkürzt werden können. Die Prozesszeiten ohne Unterbrechung sollten also möglichst lang sein.

Die Algen sollten zum Verzehr geeignet sein und daher frei von schädlichen Kontaminationen sein.

### Lösung der Aufgabe

Die Aufgabe wird gelöst mit den Merkmalen der unabhängigen Ansprüche. Vorteilhafte Ausgestaltungen der Erfindung sind in den abhängigen Ansprüchen angegeben.

Gegenstand der Erfindung ist ein Verfahren zur kontinuierlichen Algenproduktion in einem Nährmedium innerhalb eines Reaktors, mit den Schritten
1. Inokulation eines Nährmediums mit Algen,
2. Durchmischung bis zum Erreichen einer konstanten Biomassekonzentration von 0,3-0,8 g/L im Nährmedium,
3. Kontinuierlicher Betrieb der Algenkultivierung in dem Reaktor unter Beibehaltung dieser Biomassekonzentration, umfassend die Schritte:
   3a. Entfernen eines Teils des Nährmediums, sowie
   3b. Abtrennung der Algen daraus, und
   3c. Hinzufügen mindestens eines Teils des in Schritt 3a. entfernten Nährmediums nach Algenabtrennung als von Algen befreites, wieder aufbereitetes Nährmedium,
   (so dass also auch die Schritte 3a., 3b. und 3c. kontinuierlich ablaufen),
   bei gleichzeitiger Regelung (im kontinuierlichen Betrieb also in Schritt 3. des Verfahrens) mindestens einer Regelgröße im kontinuierlichen Betrieb im Reaktor, ausgewählt aus Verdünnungsfaktor, eingestrahlter Lichtmenge und Temperatur, um die konstante Biomassekonzentration beizubehalten.

Der "Verdünnungsfaktor" gibt an, wie oft das Nährmedium im Reaktor pro Zeit ausgetauscht wird. Damit wird für eine Verdünnung und damit eine Verringerung der optischen Dichte der Algensuspension und damit auch der Biomassekonzentration gesorgt.

Er berechnet sich somit aus dem in Schritt 3a. entfernten Teil des Nährmediums pro Zeit. Denn das entfernte Nährmedium-Volumen wird, da die Reaktorfüllung im kontinuierlichen Betrieb konstant gehalten wird, im gleichen Volumen bei Schritt 3c wieder hinzugefügt (mindestens ein Teil als von Algen befreites Nährmedium und unter Umständen gemischt mit neuem, frischen Nährmedium). Ein Verdünnungsfaktor von 0,3-1 pro Tag bedeutet somit, dass 30% bis 100% des gesamten Reaktorinhalts pro 24 Stunden vollständig ausgetauscht werden.

Bzgl. der "eingestrahlten Lichtmenge" kann der Einfluss des Lichtes auf die Veränderung der Biomassekonzentration (oder den Trübungswert) mittels mathematischer Berechnungen ermittelt werden und die Zufuhr von Licht entsprechend (automatisch oder manuell) eingestellt werden. Damit ist ein besonders energieeffizienter Betrieb möglich.

Auch die Einstellung der "Temperatur" als Regelgröße in Abhängigkeit von der Biomassekonzentration zum Zeitpunkt der Bestimmung erlaubt eine Möglichkeit, um die erfindungsgemäße, konstante Biomassekonzentration von 0,3-0,8 g/L im Nährmedium beizubehalten.

Gegenstand der Erfindung ist darüber hinaus auch eine Anlage zur kontinuierlichen Algenproduktion in einem Nährmedium, umfassend:
- einen Reaktor,
- mit am Reaktor angeschlossenem Trübungssensor zur Überwachung der Trübung des Nährmediums im Reaktor,
- eine Algenabtrennungseinheit,
- eine Leitung vom Reaktor zur Algenabtrennungseinheit und von dort zurück zum Reaktor, und
- als Regelungseinheit, zur Regelung einer Regelgröße im Reaktor,
   eine Pumpe zur Regelung eines Verdünnungsfaktors und/oder
   eine Lichtquelle zur Regelung der Lichtmenge (welche bspw. über einen PAR-Sensor gemessen werden kann) und/oder
   eine Heiz-Kühleinheit zur Regelung der Temperatur,
   jeweils mit Steuerungseinheit zum Abgleich des Trübungssensors mit der Regelungseinheit, so dass ein Trübungswert als Sollgröße eingehalten werden kann.

Sinnvollerweise ist auch eine automatisch gesteuerte oder manuell betrieben Zufuhreinheit für frisches Nährmedium umfasst.

Gegenstand der Erfindung ist schließlich auch die Verwendung der erfindungsgemäßen Anlage zur kontinuierlichen Algenproduktion mit dem erfindungsgemäßen Verfahren.

Die Umrechnung von Biomassekonzentration (in g/L) in optische Dichte (bei 750nm) bei Algen erfolgt mittels des Faktors 1/0,86. Das heißt eine Biomassekonzentration von 0,3g/L entspricht beispielsweise einer optischen Dichte von 0,35.

Der Fachmann erkennt, welche Schritte des erfindungsgemäßen Verfahrens mit welchen Anlagenbauteilen durchgeführt werden. Ausführungen zu den einzelnen Schritten betreffen demnach in analoger Weise die Anlagenbauteile und umgekehrt. Beispielsweise gelten Ausführungen zur Algenabtrennung in Schritt 3b. des Verfahrens in analoger Weise für die Algenabtrennungseinheit der erfindungsgemäßen Anlage und umgekehrt.

"Algen" im Sinne der Erfindung sind Mikroalgen, wie beispielsweise *Chlorella Vulgaris,* und auch Cyanobakterien, die Photosynthese betreiben, wie bspw. *Arthrospira Platensis.*

Das Entfernen eines Teils des Nährmediums in Schritt 3a. kann in der Art eines Ausleitens des entfernten Mediums aus dem Reaktor erfolgen oder aber auch nur durch Abzweigung innerhalb des Reaktors in der Art eines Bypasses, wobei die Algenabtrennung dann in diesem Reaktor-Bypass stattfinden würde.

### Zum Reaktor:

Als Reaktor kommen verschiedene Reaktortypen, wie beispielsweise Photobioreaktor, Plattenreaktor oder auch offene Becken (Pond-Reaktoren) in Frage.

Die erfindungsgemäße Inokulation des Nährmediums (Schritt 1.) und das Durchmischen (Schritt 2.) können sowohl kontinuierlich bereits in dem Reaktor oder auch diskontinuierliche im Batchbetrieb/Satzbetrieb stattfinden. Im Falle des Batchbetriebes weiß der Fachmann, dass dann das Nährmedium mit den Algen mit der konstanten Trübung in den erfindungsgemäßen Reaktor für Schritt 3. Überführt werden muss. Aufgrund der dabei bestehenden Kontaminationsgefahr wird bevorzugt mindestens Schritt 2. bereits im Reaktor (des Schritts 3.) durchgeführt, besonders bevorzugt auch Schritt 1.

Erfindungsgemäß finden sinnvollerweise die Schritte 3a, 3b. und 3c. kontinuierlich statt, wobei "kontinuierlicher Betrieb" im Sinne der Erfindung auch kurze Pausen von maximal 30 Minuten bei den Schritten 3a. bis 3c. umfasst - solange dir Biomassekonzentration im Nährmedium erfindungsgemäß konstant gehalten wird. Kern ist jedenfalls auch, dass ein Teil des (in Schritt 3a.) entfernten Nährmediums nach Abtrennung der Algen im Kreislauf gefahren wird und (als Schritt 3c.) mindestens teilweise zurück in den Reaktor gegeben wird, was einem kontinuierlichen Betrieb entspricht.

Gegenstand der Erfindung ist schließlich auch die Verwendung der erfindungsgemäßen Anlage zur kontinuierlichen Algenproduktion im Turbidostat-Betrieb in einem Nährmedium, insbesondere die Verwendung im erfindungsgemäßen Verfahren.

Angaben "ein", "eine" oder "einer" bedeuten im Sinne der Erfindung, dass auch mehr als genau eins der betreffenden Merkmale vorhanden sein kann, außer es ist explizit anders angegeben.

Vorteil der Erfindung ist, dass damit ein Betrieb im großtechnischen Maßstab ermöglicht wird, d.h. mit >100L Volumen. Es besteht keine bisher beobachtbare Maximalbegrenzung der Kultivierungsdauer hinsichtlich des Wachstums der Alge. Lediglich Wartungsarbeiten begrenzen die Dauer der kontinuierlichen Algenproduktion.

Die volumetrische Produktivität der Algen ist bei Überwachung der Regelgröße im kontinuierlichen Betrieb als Soll-Größe höher als im Batch-Betrieb.

### Bevorzugte Ausführungsformen

In einer bevorzugten Ausführungsform des erfindungsgemäßen Verfahrens wird die Durchmischung in Schritt 2. durchgeführt bis zum Erreichen eines konstanten Trübungswertes von 0,4-0,9 optischer Dichte bei einer Wellenlänge des eingestrahlten Lichts von 750nm im Nährmedium und dieser Trübungswert wird während des kontinuierlichen Betriebs in Schritt 3. in diesem Bereich konstant gehalten wird. Besonders bevorzugt ist ein konstanter Trübungswert von 0,5-0,9 optischer Dichte.

Diese Art des kontinuierlichen Betriebs unter Beibehaltung des konstanten Trübungswertes im Nährmedium wird Turbidostat-Betrieb genannt. "Konstanter Trübungswert" bedeutet, dass dieser nicht weiter als ±0,2 optische Dichte bei 750nm um einen Wert schwankt (innerhalb eines angemessenen Zeitraums von 5 maximal 5 Minuten). Insbesondere bewegt sich der konstante Trübungswert sogar in einem Bereich von ± 10%.

### Zur Trübung:

Der Trübungssensor kann bevorzugt ein JUMO ecoline NTU sein mit Messbereich zwischen 400 und 1500 NTU. Er ist so am Reaktor angebracht, dass damit die Trübung der Flüssigkeit im Reaktor gemessen werden kann.

Die Trübungsmessung erfolgt nach DIN EN ISO 7027. Die Überwachung der Trübung erfolgt bevorzugt bei 750nm (±50nm). Es hat sich jedenfalls herausgestellt, dass eine Trübungsmessung bei dieser Wellenlänge von 750nm (±50nm) besonders geeignet ist, um die tatsächliche Algenkonzentration abzubilden, unabhängig von dem verwendeten Nährmedium bzw. dessen gewöhnlicher Inhaltsstoffe. Denn bei eingestrahltem Licht von 750nm (±50nm) wird dieses nicht von der Alge absorbiert sondern reflektiert, so dass eine Trübungsmessung fehlerfrei möglich ist. Der Fehler bei der Bestimmung der tatsächlichen Algenkonzentration über die Trübung im Reaktor ist sehr gering. In Folge ist die volumetrische Produktivität der Algen konstanter und besser reproduzierbar, was für eine großtechnische Produktion wichtig ist. Der Trübungssensor bzw. dessen Messwerte werden je nach eingesetzter Algenart mittels Kalibrierkurven zu Werten Optischer Dichten bzw. Biomassekonzentrationen umgerechnet.

Der Trübungssensor kann außen oder innen im Reaktor angebracht werden. Ist er außen angebracht, ist der Reaktor sinnvollerweise aus einem transparenten Material gefertigt, so dass der Trübungssensor die Trübung im Innern des Reaktors erfassen kann. Da allerdings bei der Algenproduktion bekanntermaßen Licht eingestrahlt werden muss, ist das Reaktormaterial zumindest von Teilbereichen des Reaktors Glas, so dass eine Anbringung des Trübungssensors außen in Frage kommt.

In einer bevorzugten Ausführungsform des Verfahrens wird der kontinuierliche Betrieb in Schritt 3. für mindestens 3 Tage ununterbrochen durchgeführt.

In einer ebenfalls bevorzugten Ausführungsform des erfindungsgemäßen Verfahrens wird bei der Regelung der Regelgröße der eingestrahlten Lichtmenge in Schritt 3. Licht eingestrahlt, dessen Wellenlängenspektrum zu ≥12% bei 400-500nm und zu ≥60% bei 600-700nm liegt. Vorteilhaft kommt es dabei zu wenig Photoinhibition.

In einer bevorzugten Ausführung des Verfahrens werden als Regelgrößen in Schritt 3. der Verdünnungsfaktor und die eingestrahlte Lichtmenge und die Temperatur geregelt. Dementsprechend würde auch die erfindungsgemäße Anlage in solch einer Ausführung als Regelungseinheiten sowohl Pumpe, als auch Lichtquelle und Heiz-Kühleinheit aufweisen.

In einer bevorzugten Ausführung der Erfindung umfasst die Algenabtrennung (bzw. entsprechend die Algenabtrennungseinheit) eine Elektrokoagulation (bzw. eine Elektrokoagulationseinheit) zur Aufkonzentration der Algen.

"Elektrokoagulation" im Sinne der Erfindung bedeutet, dass die Algen im Nährmedium ein elektrisches Feld (innerhalb der Elektrokoagulationseinheit) durchlaufen und aufgrund ihrer Oberflächenspannungsverringerung zu größeren Partikeln verklumpen und zu Boden sinken (sedimentieren) oder sogar (je nach Spannung durch Zersetzung von Wasser und Bildung von Mikroblasen aus H₂ und O₂) an die Oberfläche steigen. Der Fachmann kennt Methoden, um die oben schwimmenden oder unten sedimentierten Algenpartikel von der Flüssigkeit zu separieren.

Das heißt die Elektrokoagulation dient der Flotation, der Sedimentation oder auch lediglich der Partikelvergrößerung. Eine sich anschließende Separierung findet mit bekannten Methoden statt, beispielsweise Filtration (wie Cross-Flow-Filtration oder Rotating-Disk-Filtration, Vibrationssieb, Schrägfilter) oder Hydrozyklon. "Hydrozyklone" sind Fliehkraftabscheider für Flüssiggemische. Mit Hydrozyklonen werden in Suspensionen enthaltene Feststoffpartikel (wie bspw. Algen) abgetrennt oder klassiert. Ebenso können auch Emulsionen, wie z. B. Öl-Wasser-Gemische, getrennt werden. Bei der Erfindung beispielsweise können Algen als separater Strom abgetrennt werden, beispielsweise über eine Schnecke im Hydrozyklon.

Sinnvollerweist werden die Algenteilchen im entfernten Teil des Nährmediums (siehe Schritt 3a.) so durch die Elektrokoagulationseinheit geleitet, dass sich jedes Algenteilchen mindestens 0,2 min im elektrischen Feld der Elektrokoagulationseinheit befindet.

Die Wahl von Elektrokoagulation für die Abtrennung der Algen (auch Ernte oder Downstream genannt) hat den Vorteil, dass keine Unterbrechungen zur Reinigung oder Entleerung nötig sind. Denn entweder verklumpen die Algen dadurch und ein späterer Filter verstopft nicht mehr so stark (aufgrund der größeren Partikelgrößen der Algen). Oder aber die Algen sedimentieren und können bedeckt von Flüssigkeit bspw. mittels Schieber entfernt werden. Es ist bei gezielter Hydrolyse des Wassers unter Blasenbildung aber auch möglich, eine Flotation herbeizuführen, so dass die Algen mit den Blasen nach oben steigen und dort unterbrechungsfrei abgenommen werden können.

Diese Ausführungsform ermöglicht somit kürzere Prozesslaufzeiten, da auf Wartungen bspw. aufgrund verstopfter Filter verzichtet werden kann. Besonders bevorzugt ist somit, dass die Algenabtrennung sowohl Elektrokoagulation(-seinheit) als auch eine Filtration(-seinheit) oder ein Hydrozyklon umfasst.

Jedenfalls kommt es vorteilhaft bei der Elektrokoagulation zu einer kontinuierlichen Vorkonzentration aufgrund Flockung gemäß der Oberflächenspannung. D.h. bei einer sich anschließenden Separierung treten wesentlich weniger Verstopfungen bspw. des Filters auf bzw. eine Trennung mittels Hydrozyklon wird wesentlich erleichtert und effektiviert.

Das heißt vorteilhaft kann durch die Elektrokoagulation im Separierungsschritt auf grobporigere Membranen, Siebe bzw. Filter zurückgegriffen werden (mit Porengrößen von bspw. 50-100µm). Dadurch wird der Wartungsaufwand wesentlich verringert.

Im Falle eines Hydrozyklons sind vorteilhaft größere Fließgeschwindigkeiten möglich, als es ohne Elektrokoagulation der Fall wäre.

Zusammenfassend wird dadurch der Durchsatz bei der Algenabtrennung, d.h. im sogenannten Downstreambereich, vergrößert.

Der Aufbau ist entsprechend einfach und erlaubt somit auch im großtechnischen Maßstab eine kostengünstige Produktion der Algen.

Wie bereits beschrieben sind wegen der Elektrokoagulation die abzutrennenden Algen größer und es kommt zu geringerem Wartungsaufwand (weniger Filterverstopfung) oder leichterer

Abtrennung. Jedenfalls wird die Anlage weniger oft oder (während der Produktion) sogar gar nicht mehr zu öffnen sein, so dass die Gefahr von Verunreinigungen maßgeblich sinkt. Die Algen sind somit vorteilhaft zum Verzehr geeignet.

In einer bevorzugten Ausführungsform des erfindungsgemäßen Verfahrens ist die Regelgröße, die in Schritt 3. im Reaktor geregelt wird,
ein Verdünnungsfaktor von 0,15-1,0 pro Tag und/oder
eine eingestrahlte Lichtmenge von 100-400 µmol/m²s und/oder
eine Temperatur von 15-40°C.

Beim Verdünnungsfaktor sind ganz besonders 0,28-1,0 pro Tag (oder sogar 0,28-0,30 pro Tag) und bei der Temperatur 15-34°C bevorzugt.

Vorteilhaft kommt es zu wenig Photoinhibition. Es handelt es sich um den energetisch besten Betriebspunkt. Eine mehrdimensionale Regelung mittels maschineller Lernalgorithmen ist möglich.

In einer bevorzugten Ausführungsform der Erfindung ist der Reaktor ein Rohrreaktor (z.B. modular aufgebaut) mit >6.000 L Kultivierungsvolumen, insbesondere sogar 8.000 bis >10.000 L.

In einer bevorzugten Ausführungsform der Erfindung ist eine Lichtquelle als regelbares Beleuchtungssystem umfasst, welche als alleinige Beleuchtung oder additiv zur solaren Einstrahlung in der Intensität automatisch oder manuell gesteuert werden kann (Einstellungen: ein, aus oder heller oder dunkler). Ganz besonders bevorzugt kann die Lichtmenge über einen PAR Sensor gemessen werden.

In einer bevorzugten Ausführungsform der Erfindung handelt es sich bei den zu produzierenden Algen um "Limnospira maxima".

In einer Ausführungsform der Erfindung handelt es sich bei dem Reaktor um ein offenes Becken (d.h. um einen offenen Pond) oder einem Rohrreaktor.

In einer bevorzugten Ausführung handelt es sich bei dem Reaktor um einen Rohrreaktor. Dieser hat besonders bevorzugt eine mäandrierende Form mit dreidimensional maximal 7 x maximal 35 angeordneten Schlaufen (Schlaufen wie beispielsweise in EP 2 486 790 A1 dargestellt). Vorteil dieser Ausführung ist, dass Sie aus Schlaufen gebildete Rohreinheit in einen handelsüblichen Container passt. Besonders bevorzugt sind 6x30 Schlaufen. Eine leichte Parallelisierung solcher aus Schlaufen gebildeter Rohreinheiten ist möglich, containerweise.

In einer bevorzugten Ausführungsform ist der Reaktor ein Rohrreaktor und die Elektrokoagulationseinheit ist ein Teilabschnitt des Rohrreaktors, wie beispielsweise ein Bypass, d.h. eine Abzweigung des Rohrreaktors, die für die Elektrokoagulation ausgestaltet ist.

In einer bevorzugten Ausführung der Erfindung ist die Elektrokoagulationseinheit eine röhrenförmige Einheit. Ganz besonders bevorzugt beträgt die Länge der Elektrokoagulationseinheit dabei 50 cm (±5cm).

In einer bevorzugten Ausführung der Erfindung umfasst die Abtrennungseinheit
- eine Elektrokoagulationseinheit innerhalb des Reaktors zur Verklumpung der Algen, und
- eine Separierungseinheit, ausgewählt aus einer Filtrationseinheit und einem Hydrozyklon,
wobei die vom Reaktor in Algenfließrichtung kommende Leitung zuerst zur Elektrokoagulationseinheit und danach zur Separierungseinheit (Filter oder Hydrozyklon) führt. Im erfindungsgemäßen Verfahren würde sich bei der Algenabtrennung in Schritt 3b. mittels Elektrokoagulation dann ein Schritt der Separierung der Algen mittels Filtration (mittels Filter) oder Hydrozyklon anschließen.

Besonders bevorzugt findet die Separierung in der Separierungseinheit (als Teil der Abtrennung/Abtrennungseinheit) mittels Filter statt, wobei dieser eine Porengröße im Bereich 2-100µm hat (auch 50-100µm ist möglich). Vorteilhaft eignet sich diese Porengröße besonders für die Erfindung, bei der mittels Elektrokoagulation die Partikelgröße der Algen erhöht wurde.

In einer weiteren bevorzugten Ausführung der Erfindung ist der Reaktor ein Rohrreaktor und es umfasst die Abtrennungseinheit (der erfindungsgemäßen Anlage):
- eine Elektrokoagulationseinheit innerhalb des Rohrreaktors (d.h. des Rohrsystems des Reaktors) zur Verklumpung der Algen, und
- eine Separierungseinheit, ausgewählt aus einer Filtrationseinheit und einem Hydrozyklon,

wobei die vom Reaktor in Algenfließrichtung kommende Leitung zuerst zur Elektrokoagulationseinheit und danach zur Separierungseinheit (Filter oder Hydrozyklon) führt, und
wobei die Algenabtrennungseinheit als geschlossene Umgebung ausgestaltet ist, so dass die Algen unter Luftausschluss abgetrennt werden können.

Dies entspricht erkennbar der Ausführung des erfindungsgemäßen Verfahrens, bei der der Reaktor ein Rohrreaktor ist, und
die Algenabtrennung in Schritt 3b. mittels Elektrokoagulation innerhalb des Rohrreaktors zur Verklumpung mit sich anschließender Separierung mittels Filter oder Hydrozyklon stattfindet, und
wobei sowohl die Elektrokoagulation als auch die sich anschließende Separierung in einer geschlossenen Umgebung stattfindet (optional unter Luftausschluss) - d.h. die ganze Algenabtrennung in der erfindungsgemäßen Algenabtrennungseinheit findet in einer geschlossenen Umgebung statt.

Vorteil dieser beiden ähnlichen Ausführungen ist, dass der Aufbau sehr kompakt und einfach ist.

Denn mit einer größeren Partikelgröße (aufgrund der Verklumpung bei der Elektrokoagulation) können die Algenpartikel nicht so weit in die Filtermembranporen eindringen und diese zusetzen. Im Hydrozyklon ist eine Abtrennung von der Flüssigkeit mittels Fliehkraft wesentlich genauer bei einer größeren Partikelgröße der abzutrennenden Algenpartikel möglich. Es können größere Filtermembranen eingesetzt werden bzw. die Fließgeschwindigkeit bei Hydrozyklonen kann erhöht werden.

Die Anlage in diesen beiden Ausführungen bedarf weniger Ventile, Abzweigungen und sonstiger Problemquellen. Noch dazu sind die Algen aufgrund der geschlossenen Umgebung vor Verunreinigungen geschützt sind und somit zum Verzehr geeignet sind. Denn es kann zu keinerlei Kontamination der produzierten Algen kommen. Bei besonders empfindlichen Algenarten wäre es damit auch möglich, unter Schutzgasatmosphäre die Algen abzutrennen, so dass diese vor ungewollter Oxidation geschützt sind. Die Algen jedenfalls sind vorteilhaft zum Verzehr geeignet.

Es soll auch umfasst sein, dass die Separierungseinheit der Algenabtrennungseinheit sowohl Filter als auch Hydrozyklon aufweist. Dabei befinden sich sinnvollerweise zwischen diesen beiden eine Leitung, welche das Nährmedium vom einen zum anderen Bauteil befördert.

Ganz besonders bevorzugt findet in diesen Ausführungen mit geschlossener Umgebung eine Begasung mit steril filtrierter Luft statt, besonders bevorzugt mittels 0,2µm Sterilfilter filtriert. Je nach Algenstamm kann diese mit CO₂ angereichert werden. Dabei hätte eine Anlage ein entsprechendes Ventil zum Mischen von Luft und CO₂.

In einer Variante dieser beiden Ausführungen mit Separierungseinheit ausgewählt aus Filter, kann dieser Filter ein Schwerkraftfilter, ein Trommelfilter oder ein Vibrationssieb sein.

In einer bevorzugten Ausführung wird das künstlich erzeugte Licht durch LEDs in einem von den Algen absorbierbaren, photosyntheseaktiven Spektrum erzeugt, die automatisch oder manuell entsprechend der Steuerungsvorgaben ein- oder aus- bzw. in Ihrer Intensität heller oder dunkler geschaltet werden können. Das kann auch einzelne spektrale Bereiche des LED Spektrums betreffen. Vorteilhaft sind LEDs energiesparsam.

In einer ebenfalls bevorzugten Ausführung der Erfindung umfasst die erfindungsgemäße Anlage einen Regler, eine Steuereinheit und ein Ventil, zur Anpassung der Menge des aus dem Reaktor zu entfernenden Nährmediums, so dass ein Trübungswert (im Reaktor in dem Nährmedium) als Sollgröße eingehalten werden kann.

In einer ebenfalls bevorzugten Ausführungsform der Erfindung ist bei der Elektrokoagulation (bzw. der Elektrokoagulationseinheit) die Kathode aus Edelstahl und/oder die Anode aus Mg, Fe oder Graphit. Ganz besonders bevorzugt ist die Anode aus Magnesium oder Graphit.

Die Elektrokoagulationseinheit umfasst in einer ebenso bevorzugten Ausführungsform ein Rohr als Kathode, insbesondere einen Edelstahlrohrabschnitt, und einen Anoden-Stab, insbesondere einen Anoden-Stab aus Magnesium. Der Anoden-Stab kann aber auch als inerte Anode aus Graphit gefertigt sein.

In einer besonders bevorzugten Ausführungsform der Erfindung ist bei der Elektrokoagulationseinheit (bzw. der Elektrokoagulation) die Kathode aus Edelstahl und die Anode eine Opferanode aus Mg. Vorteilhaft sind damit die mit der Erfindung herstellbaren Algen zum Verzehr geeignet. Denn mit dieser Opferanode werden Ionen freigesetzt und die Algenpartikel sedimentieren und bleiben unter der Flüssigkeit vor Verunreinigungen und Oxidation geschützt.

Bevorzugt umfasst die Elektrokoagulationseinheit in einer Ausführungsform ein Kunststoff-Schutzrohr, welches sowohl Anode als auch Kathode einschließt.

In einer Ausführungsform, bei der in Schritt 3c. auch zusätzlich frisches Nährmedium dem Reaktor hinzugefügt wird (im kontinuierlichen Betrieb) mündet sinnvollerweise eine Frischmediumleitung von einem Nährmedium-Vorratsbehälter (für frisches Nährmedium) in die erfindungsgemäße Leitung von der Algenabtrennungseinheit zurück zum Reaktor, d.h. es ist auch solch eine Frischmediumleitung umfasst.

Bevorzugt werden in dem Prozess alle Medienströme in allen Prozesschritten in einem geschlossenen System gefahren bzw. vor Eintrag von äußeren Verschmutzungen und Kontaminationen geeignet geschützt.

In einer bevorzugten Ausführung des erfindungsgemäßen Verfahrens liegt der konstante Trübungswert in Schritt 2. bei 0,5-0,9 optischer Dichte bei einer Wellenlänge des eingestrahlten Lichts von 750nm.

Dies entspricht einer (Algen-)Biotrockenmasse von 0,5-0,7 g/L bezogen auf das Volumen des Nährmediums, beispielsweise bei der Alge *Spirulina limnospira maxima.* Dies ermöglicht vorteilhaft ein effektiveres Verfahren, d.h. die Volumen-Zeit-Ausbeute des Verfahrens ist aufgrund der hohen volumetrischen Produktivität der Algen in diesem sehr schmalen Bereich sehr hoch.

Darüber hinaus hat sich gezeigt, dass die hohe volumetrische Produktivität insbesondere bei dieser konstanten Algen-Konzentrationen von 0,5-0,7 g/L auftritt, wobei dieser Bereich mittels Trübung von 0,5-0,9 optischer Dichte bei 750nm Wellenlänge des Lichtes leicht eingestellt und kontinuierlich überwacht werden kann.

Ganz besonders bevorzugt liegt der konstante Trübungswert sogar bei 0,6-0,8 optischer Dichte bei 750nm.

Es ist in einer Ausführung des erfindungsgemäßen Verfahrens möglich, dass die in Schritt 3a. (das ist die kontinuierliche Entfernung eines Teils des Nährmediums) die Menge des kontinuierlich entfernten Nährmediums 8-12 Vol-% pro h beträgt (ganz besonders bevorzugt 10 Vol-%/h), bezogen auf das Gesamtvolumen des Nährmediums im Reaktor. Ganz besonders bevorzugt liegt dann der konstante Trübungswert bei 0,5-0,9 optischer Dichte bei 750nm. Denn Vorteil dieser Kombination ist, dass im Bereich dieses (Grenz-)Trübungswertes von 0,5-0,9 optischer Dichte ein Maximum der volumetrischen Produktivität der Algen (d.h. ein Maximum des Algenwachstums) erreicht wird und dass bei dieser Menge kontinuierlich ausgetauschten Mediums gleichzeitig eine konstante Nährstoffkonzentration erreicht wird, d.h. dass neben dem Turbidostat-Betrieb also auch ein Chemostat-Betrieb vorliegen kann.

Besonders bevorzugt wird der gesamte Teil des Nähmediums (in Schritt 3b.) von Algen befreit und komplett (in Schritt 3c.) in den Reaktor zurückgeführt, ohne frisches Nährmedium zuzumischen. Dabei wird das aufbereitete Nährmedium durch geeignete Behandlungsschritte (die der Fachmann kennt, wie z.B. Ultrafiltration, UV-Behandlung) von Kontaminanten desinfiziert.

Sinnvollerweise jedenfalls ist das Volumen entfernten Mediums und das Volumen hinzugefügten Mediums annähernd gleich, so dass der Füllstand des Reaktors weitestgehend konstant bleibt.

Denn dies ist zu trennen von der Frage, ob das in Schritt 3c hinzugefügte Nährmedium ausschließlich wiederverwendetes Nährmedium oder auch frisches Nährmedium ist.

In einer bevorzugten Ausführungsform des erfindungsgemäßen Verfahrens ist der Reaktor ein Rohrreaktor und die Strömungsgeschwindigkeit durch den Rohrreaktor in Schritt 3 liegt bei >0,3 bis 0,8 m/s, oder sogar >0,5 bis 0,8 m/s. Dies ist vorteilhaft, um unter Konstanthalten dieser Strömungsgeschwindigkeit auch die Elektrokoagulation effektiv betreiben zu können.

In einer ebenfalls bevorzugten Ausführungsform des erfindungsgemäßen Verfahrens liegt der erfindungsgemäß konstante Trübungswert bei 0,6-0,8 optischer Dichte bei 750nm, und die Konzentration von Nährstoffen im in Schritt 1. verwendeten Nährmedium bei mindestens 6g/L NaHCO₃ und bei 0,23g/L NaNO₃ ±15%. Ganz besonders bevorzugt ist bei diesem konstanten Trübungswert das NaHCO₃ mit 6,8-9,2 g/L (oder auch 8g/L) im Nährmedium enthalten und/oder das NaNO₃ mit 0,23g/L enthalten.

In einer weiteren bevorzugten Ausführungsform des erfindungsgemäßen Verfahrens findet die Algenabtrennung in Schritt 3b. mittels Elektrokoagulation in einem Rohrabschnitt einer Elektrokoagulationseinheit von 40-60cm Länge (des Rohrabschnitts) und mit einer Fließgeschwindigkeit (innerhalb der Elektrokoagulationseinheit) von 0,007-0,03m/s statt. Vorteilhaft eignen sich diese Parameter besonders gut für eine effektive Verklumpung mittels Elektrokoagulation für viele großtechnisch ausgelegte Varianten der Erfindung. Der Rohrabschnitt hat ganz besonders bevorzugt einen Innendurchmesser der röhrenförmigen Kathode von 50mm±2mm, wobei die stabförmige Anode einen Außendurchmesser von 21mm±2mm aufweist.

In einer ebenfalls bevorzugten Ausführung findet die Algenabtrennung in Schritt 3b. mittels Elektrokoagulation bei einer Spannung von 12-36 V (besonders bevorzugt 12-18V) und mit einer Opferanode aus Magnesium oder einer inerten Anode aus Graphit statt. Vorteilhaft kommt es dabei nicht bereits zu einer Mikroblasenbildung (aufgrund Elektrolyse von Wasser) und dennoch ist die Elektrokoagulation effektiv genug durchführbar. Das heißt. Algen steigen nicht nach oben und können (bedeckt von Flüssigkeit) ohne die Gefahr von Verunreinigungen am Boden des Reaktors bzw. der Elektrokoagulationseinheit abgetrennt werden. Bevorzugt handelt es sich um eine röhrenförmige Elektrokoagulationseinheit, wobei die Anode als Stab einen Außendurchmesser von 21mm und die Kathode als Rohr einen Innendurchmesser von 50mm aufweisen.

Die Temperatur bei der Kultivierung (insbesondere im kontinuierlichen Betrieb in Schritt 3.) liegt bevorzugt bei 30°C±2°C. Vorteilhaft liegt dabei die Wachstumsrate in einem günstigen Bereich.

In einer bevorzugten Ausführungsform des Verfahrens mit einem Photobioreaktor als Reaktor liegen folgende Parameter vor:
- Verdünnungsfaktor (Durchflussrate durch den Photobioreaktor): 0,34 d⁻¹, und/oder
- Biotrockenmassekonzentration: 0,755 g L⁻¹, und/oder
- Phycocyaningehalt: 13 %, und/oder
- Produktivität: 0,26 g L⁻¹ d⁻¹,
jeweils in Bereichsgrenzen von ±10% des jeweiligen Wertes.

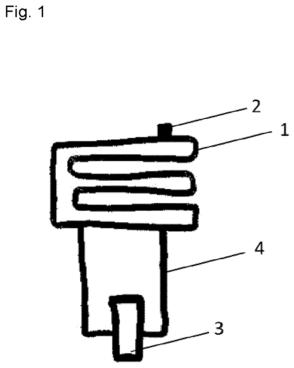

Fig. 1 zeigt eine skizzenhafte Ausführung der Erfindung, bei der der Trübungssensor außen am Reaktor angebracht worden ist, wobei der Reaktor sinnvollerweise aus einem transparenten Material, wie Glas, gefertigt wurde und als Rohrreaktor ausgestaltet ist.

Fig. 2 zeigt die Ergebnisse eines kontinuierlichen Betriebs eines 0,5 L (Labor-) Photobioreaktors.

Für die Realisierung der Erfindung ist es auch zweckmäßig, die vorbeschriebenen erfindungsgemäßen Ausgestaltungen, Ausführungsformen und Merkmale der Ansprüche miteinander zu kombinieren. Nachfolgend soll die Erfindung anhand eines Ausführungsbeispiels eingehender erläutert werden, ohne auf die Merkmale beschränkt zu sein.

### Ausführungsbeispiele

### Ausführungsbeispiel 1:

Die Kultivierung der Algenart *Limnospira maxima* wird im Satzbetrieb (= Batchbetrieb) angefahren, bis ein konstanter Trübungswert (hier Trübungswert von 0,5-0,9 optische Dichte bei 750nm) erreicht ist.

### Zur Trübungsmessung nach DIN EN ISO 7027:

Der Trübungssensor 2 ist ein JUMO ecoline NTU mit Messbereich zwischen 400 und 1500 NTU. Das Messprinzip des Trübungssensors 2 JUMO ecoLine NTU basiert auf der Infrarotlichtmessung nach dem 90°-Streulichtverfahren.

Als Reaktor 1 wird ein Rohrreaktor aus Schlaufen eines Rohres wie in EP 2 486 790 A1 verwendet. Dabei wurden in horizontaler Richtung nebeneinander 6 Schlaufen und in vertikaler Richtung 30 Schlaufen übereinander angeordnet.

Das Wachstumsmaximum, also die maximale volumetrische Produktivität der Algen, wurde bei ca. 0,6 und 0,8 optischer Dichte bei 750nm eingestellt. Dies entspricht einer Biotrockenmasse von etwa 0,5 - 0,7 g/l.

Es findet eine Begasung mit steril filtrierter Luft statt, die zeitweise mit CO₂ angereichert wurde.

Die Nährstoffkonzentration im kontinuierlichen Betrieb beträgt im Reaktor: NaHCO₃ 8g/l; NaNO₃ 230mg/l; NPK-Dünger 0,5ml/l.

Anschließend erfolgt der Übergang in den kontinuierlichen Betrieb, bei dem recyceltes (= von Algen befreites Nährmedium) und ggf. auch frisces Medium zugeführt und Algensuspension abgeführt wird.

Zur Elektrokoagulation:
Die Abtrennung erfolgt zum Einen mittels Elektrokoagulation in einer Elektrokoagulationseinheit 3 mit einer Länge von ca. 500mm; Fließgeschwindigkeit 0,007 - 0,03 m/s; Spannung ca. 12 - 18 V; Mindestverweilzeit einzelner Algen im elektrischen Feld der Elektrokoagulationseinheit 0,2 min.

Das Elektromodul der Elektrokoagulationseinheit 3 verwendet Standard-Anoden aus dem Heizkessel-Bau und der Galvanik. Im Anschluss wurde mittels eines 50µm-Poren-Filters filtriert und die Algen getrocknet.

### Ausführungsbeispiel 2:

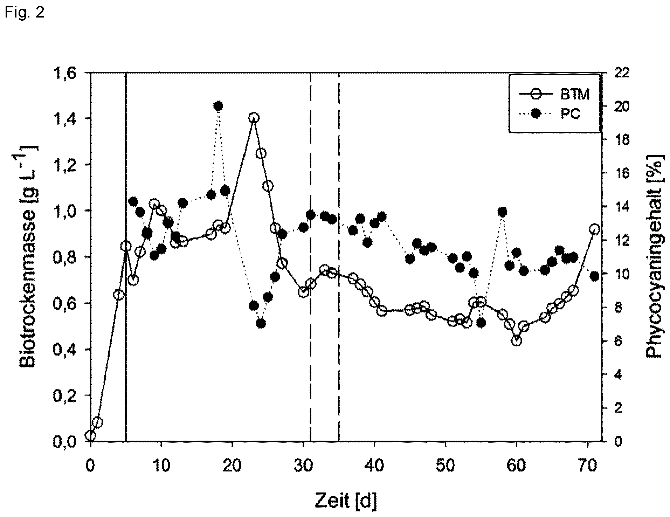

Es wurde für den Stamm *A. platensis* CCALA ein 0,5 L Photobioreaktorsystem im kontinuierlichen Betrieb betrieben (T = 30°C, initiale Photonenflussdichte I₀ = 150 µmol m⁻² s⁻¹). Im Prozessverlauf wurde die Durchflussrate im Bereich von 0,1 - 0,34 d⁻¹ variiert. Die Ergebnisse der Biomasseproduktion (BTM) sowie des zellulären Phycocyaningehaltes (PC) sind in Fig. 2 dargestellt.

Der optimale Arbeitspunkt des kontinuierlichen Systems auf Basis der Biomassebildung (Quantität der Biomassebildung) sowie eines maximalen Phycocyaningehaltes (Qualität der Biomasse) ist in Fig. 2 markiert. Der Arbeitspunkt kennzeichnet sich durch folgende Konfiguration:
- Durchflussrate: 0,34 d⁻¹
- Biotrockenmassekonzentration: 0,755 g L⁻¹
- Phycocyaningehalt: 13 %
- Produktivität: 0,26 g L⁻¹ d⁻¹.

### Ausführungsbeispiel 3: Vergleich zum Batch-Betrieb

Für den Performance-Vergleich von Satz- zu kontinuierlichem Betrieb der Algenanlage wurden beiden Betriebsmodi in der Algenanlage realisiert. Basierend auf den experimentellen Daten erfolgt der Vergleich der Prozessparameter vom 100 L - 10.000 L Maßstab in den folgenden Tabellen.

| Rechnung 1 Woche - **Volumen 100 L** | | | |
|---|---|---|---|
| **Kontinuierlich** | | **Referenz (batch)** | |
| Dauer [d] | | 7 | |
| Trockenmasse [kg] | 0,00059 | | 0,0014 |
| Verdünnungsfaktor D [d⁻¹] | 0,29 | | 0 |
| Startvolumen [L] | 100,00 | | 100 |
| Durchsatz pro Tag [L] | 29,00 | | 0 |
| Durchsatz im Zeitraum [L] | 203,00 | | 0 |
| Gesamtvolumen [L] | 303,00 | | 100 |

| **Produzierte Biomasse [kg]** | **0,18** | **0,13** |
|---|---|---|
| Prozentuale Steigerung zur Referenz [%] | 30,08 | |
| | | |

| **Volumen 10.000 L** | | | |
|---|---|---|---|
| Kontinuierlich | | Referenz (batch) | |
| Dauer [d] | | 7 | |
| Trockenmasse [kg] | 0,00059 | | 0,00125 |
| Verdünnungsfaktor D [d⁻¹] | 0,29 | | 0 |
| Startvolumen [L] | 10000,00 | | 10000 |
| Durchsatz pro Tag [L] | 2900,00 | | 0 |
| Durchsatz im Zeitraum [L] | 20300,00 | | 0 |
| Gesamtvolumen | 30300,00 | | 10000 |

| | | |
|---|---|---|
| **Produzierte Biomasse [kg]** | **17,88** | **12,50** |
| Prozentuale Steigerung zur Referenz [%] | 30,08 | |

| Rechnung 1 Monat - Volumen 100 L | | |
|---|---|---|
| Kontinuierlich | | Referenz (batch) |
| Dauer [d] | 30 | 26 |
| Trockenmasse [kg] | 0,00059 | 0,00125 |
| Verdünnungsfaktor D [d⁻¹] | 0,29 | 0 |
| Startvolumen [L] | 100,00 | 100 |
| Durchsatz pro Tag [L] | 29,00 | 0 |
| Durchsatz im Zeitraum [L] | 870,00 | 0 |
| Gesamtvolumen [L] | 970,00 | 240 |

| | | |
|---|---|---|
| Produzierte Biomasse [kg] | 0,57 | 0,28 |
| Prozentuale Steigerung zur Referenz [%] | 207,36 | |

| Volumen 10.000 L | | |
|---|---|---|
| Kontinuierlich | | Referenz (batch) |
| Dauer [d] | 30 | 26 |
| Trockenmasse [kg] | 0,00059 | 0,00125 |
| Verdünnungsfaktor D [d⁻¹] | 0,29 | 0 |
| Startvolumen [L] | 6000,00 | 10000 |
| Durchsatz pro Tag [L] | 1740,00 | 0 |

| | | |
|---|---|---|
| Durchsatz im Zeitraum [L] | 52200,00 | 0 |
| Gesamtvolumen | 58200,00 | 24000 |
| | | |
| Produzierte Biomasse [kg] | 57,23 | 27,60 |
| Prozentuale Steigerung zur Referenz [%] | 207,36 | |

Die Ergebnisse zeigen, dass bereits nach einer Woche Betrieb der kontinuierliche Betrieb eine 30%-ige Steigerung der Biomasseproduktivität erzielt. Bei kontinuierlichem Betrieb über einen Monat kann die Biomasseproduktion in etwa verdoppelt werden. Für die Berechnung wurden folgende Annahmen getroffen:
- Maximal drei Satzbetriebe pro Monat durch zwischengeschaltete Ernte- und Reinigungszyklen,
- Für die Produktivität wurden Resultate aus mindestens 5 Referenzversuchen herangezogen (Satzbetrieb) bzw. mindestens 5 Tage im stabilen kontinuierlichen Betriebsmodus gemittelt,
- Im Satzbetrieb müssen jeweils 20% des Volumens für Neuinokulation genutzt werden,
- Der kontinuierliche Betrieb wird während des Betriebs Reinigungszyklen unterworfen.

### Zitierte Nichtpatentliteratur:

López.Guzmann, M.; Flores-Hidalgo, M. A.; Reynoso-Cuevas, L.;
Electrocoagulation Process: An Approach to Continuous Processes, Reactors Design, Pharmaceuticals Removal, and Hybrid Systems - A Review.
Process 2021, 9, 1831.
Visigalli, S.; Barberis, M. G.; Turolla, A.; Canziani, R.; Zrimec, M. B.; Reinhardt, R.; Ficara, E. Electrocoagulation-flotation (ECF) for microalgae harvesting - A review.
Separation and Purification Technology 2021, 271, 118684.

### Bezugszeichen

- 1: Reaktor (mit Nährmedium)
- 2: Trübungssensor
- 3: Elektrokoagulationseinheit (Algenabtrennungseinheit)
- 4: Leitung zwischen Reaktor und Algenabtrennungseinheit

## Patentansprüche

1. Verfahren zur kontinuierlichen Algenproduktion in einem Nährmedium innerhalb eines Reaktors (1), mit den Schritten
1. Inokulation eines Nährmediums mit Algen,
2. Durchmischung bis zum Erreichen einer konstanten Biomassekonzentration von 0,3-0,8 g/L im Nährmedium,
3. Kontinuierlicher Betrieb der Algenkultivierung in dem Reaktor (1) unter Beibehaltung dieser Biomassekonzentration, umfassend die Schritte:
3a. Entfernen eines Teils des Nährmediums, sowie
3b. Abtrennung der Algen daraus, und
3c. Hinzufügen mindestens eines Teils des in Schritt 3a. entfernten Nährmediums nach Algenabtrennung als von Algen befreites, wieder aufbereitetes Nährmedium,
bei gleichzeitiger Regelung mindestens einer Regelgröße im kontinuierlichen Betrieb im Reaktor, ausgewählt aus Verdünnungsfaktor, eingestrahlte Lichtmenge und
Temperatur, um die konstante Biomassekonzentration beizubehalten.

2. Verfahren nach Anspruch 1, wobei die Durchmischung in Schritt 2. durchgeführt wird bis zum Erreichen eines konstanten Trübungswertes von 0,4-0,9 optischer Dichte bei einer Wellenlänge des eingestrahlten Lichts von 750nm im Nährmedium und dieser Trübungswert während des kontinuierlichen Betriebs in Schritt 3. in diesem Bereich konstant gehalten wird.

3. Verfahren nach einem der Ansprüche 1 oder 2, wobei der kontinuierliche Betrieb in Schritt 3. für mindestens 3 Tage ununterbrochen durchgeführt wird.

4. Verfahren nach einem der Ansprüche 1 bis 3, wobei bei der Regelung der Regelgröße der eingestrahlten Lichtmenge in Schritt 3. Licht eingestrahlt wird, dessen Wellenlängenspektrum zu ≥12% bei 400-500 nm und zu ≥60% bei 600-700nm liegt.

5. Verfahren nach einem der Ansprüche 1 bis 4, wobei als Regelgrößen in Schritt 3. Verdünnungsfaktor und eingestrahlte Lichtmenge und Temperatur geregelt werden

6. Verfahren nach einem der Ansprüche 1 bis 5, wobei die Algenabtrennung eine Elektrokoagulation (3) zur Aufkonzentration umfasst.

7. Verfahren nach einem der Ansprüche 1 bis 6, wobei die Regelgröße, die in Schritt 3. im Reaktor geregelt wird,
ein Verdünnungsfaktor von 0,15-1,0 pro Tag und/oder
eine eingestrahlte Lichtmenge von 100-400 µmol/m²s und/oder
eine Temperatur von 15-40°C ist.

8. Verfahren nach einem der Ansprüche 1 bis 7, wobei der Reaktor (1) ein Rohrreaktor ist, und
die Algenabtrennung in Schritt 3b. mittels Elektrokoagulation (3) innerhalb des Rohrreaktors zur Verklumpung mit sich anschließender Separierung mittels Filtration oder Hydrozyklon stattfindet, und
wobei sowohl die Elektrokoagulation (3) als auch die sich anschließende Separierung in einer geschlossenen Umgebung stattfindet.

9. Verfahren nach einem der Ansprüche 1 bis 8, wobei der Reaktor (1) ein Rohrreaktor ist und die Strömungsgeschwindigkeit durch den Rohrreaktor in Schritt 3 bei >0,3 bis 0,8 m/s liegt.

10. Verfahren nach einem der Ansprüche 1 bis 9, wobei die Algenabtrennung in Schritt 3b. mittels Elektrokoagulation in einem Rohrabschnitt einer Elektrokoagulationseinheit (3) von 40-60cm Länge und mit einer Fließgeschwindigkeit innerhalb der Elektrokoagulationseinheit von 0,007-0,03m/s stattfindet.

11. Verfahren nach einem der Ansprüche 1 bis 10, wobei die Algenabtrennung in Schritt 3b.
mittels Elektrokoagulation bei einer Spannung von 12-36 V und mit einer Opferanode aus Magnesium oder einer inerten Anode aus Graphit stattfindet.

12. Anlage zur kontinuierlichen Algenproduktion in einem Nährmedium, umfassend:
• einen Reaktor (1),
• mit am Reaktor angeschlossenem Trübungssensor (2) zur Überwachung der Trübung des Nährmediums im Reaktor,
• eine Algenabtrennungseinheit,
• eine Leitung (4) vom Reaktor zur Algenabtrennungseinheit und von dort zurück zum Reaktor, und
• als Regelungseinheit, zur Regelung einer Regelgröße im Reaktor,
eine Pumpe zur Regelung eines Verdünnungsfaktors und/oder
eine Lichtquelle zur Regelung der Lichtmenge und/oder
eine Heiz-Kühleinheit zur Regelung der Temperatur,
jeweils mit Steuerungseinheit zum Abgleich des Trübungssensors mit der Regelungseinheit, so dass ein Trübungswert als Sollgröße eingehalten werden kann.

13. Anlage nach Anspruch 12, wobei die Algenabtrennungseinheit eine Elektrokoagulationseinheit (3) umfasst.

14. Anlage nach einem der Ansprüche 12 oder 13, wobei der Reaktor (1) ein Rohrreaktor ist und die Abtrennungseinheit eine
• Elektrokoagulationseinheit (3) innerhalb des Rohrreaktors zur Verklumpung der Algen, und
• eine Separierungseinheit, ausgewählt aus einer Filtrationseinheit und einem Hydrozyklon,
umfasst, und
wobei die vom Rohrreaktor in Algenfließrichtung kommende Leitung (4) zuerst zur Elektrokoagulationseinheit (3) und danach zum Filter oder Hydrozyklon führt, und
wobei die Algenabtrennungseinheit als geschlossene Umgebung ausgestaltet ist, so dass die Algen unter Luftausschluss abgetrennt werden können.

15. Verwendung einer Anlage nach einem der Ansprüche 12 bis 14 zur kontinuierlichen Algenproduktion mit einem Verfahren nach einem der Ansprüche 1 bis 11.
